# EUROPEAN PATENT SPECIFICATION

(11) **EP 2 263 681 B1**
(45) Date of publication and mention of the grant of the patent: **17.06.2015**
(21) Application number: 09162954.3
(22) Date of filing: 17.06.2009
(51) Int. Cl.: A61K 35/22, A61P 35/00

(54) **Separation and formulation of bioactive fraction and subfraction from camel urine work as anticancer agent**
Trennung und Formulierung einer bioaktiven Fraktion und Unterfraktion aus der Kamelurinbearbeitung als Antikrebsmittel
Séparation et formulation de fraction bioactive et travail de sous-fraction d'urine de chameau en tant qu'agent anti-cancer

(43) Date of publication of application: 22.12.2010
(73) Proprietor: Khorshid, Fatin A., 21589 Jeddah (SA)
(72) Inventor: Khorshid, Fatin A., 21589 Jeddah (SA)
(74) Representative: Viering, Jentschura & Partner Patent- und Rechtsanwälte

(56) References cited:
- US-A1- 2005 100 610
- US-A1- 2005 100 621
- US-B1- 6 410 059
- AL-HARBI M M ET AL: "Effect of camel urine on the cytological and biochemical changes induced by cyclophosphamide in mice" JOURNAL OF ETHNOPHARMACOLOGY 19960705 IE, vol. 52, no. 3, 5 July 1996 (1996-07-05), pages 129-137, XP002552633 ISSN: 0378-8741
- KHORSID F A ET AL: "In vitro anticancer agent I-tissue culture study of human lung cancer cells A549 II-tissue study of mice leukemia cells L1210" INTERNATIONAL JOURNAL OF CANCER RESEARCH 2006 PK, vol. 2, no. 4, 2006, pages 330-344, XP002552634 ISSN: 1811-9727 1811-9735
- KHORSHID F: "Preclinical evaluation of PM 701 in experimental animals" INTERNATIONAL JOURNAL OF PHARMACOLOGY 2008 PK, vol. 4, no. 6, 2008, pages 443-451, XP002552635 ISSN: 1811-7775 1812-5700
- ANONYMOUS: "Camel urine a cure for cancer" MAKTOOB BUSSINESS HEALTH, [Online] - 19 May 2009 (2009-05-19) XP002552636 Retrieved from the Internet: URL:http://business.maktoob.com/2009000000 3564/0/PrintPage.htm>
- DATABASE WPI Week 200469 Thomson Scientific, London, GB; AN 2004-706723 XP002552639 & NZ 522 665 A (BIO GRAND CO LTD) 30 April 2004 (2004-04-30)
- KHORSHID, FA: "Potential anticancer natural product against human lung cancer cells" TRENDS IN MEDICAL RESEARCH, vol. 4, no. 1, 1 July 2009 (2009-07-01), pages 8-15, XP008113892
- KHORSHID, FA ET AL.,: "Cytotoxic activity of bioactive fractions from PM 701" ELECTRONIC JOURNAL OF ENVIRONMENTAL, AGRICULTURAL AND FOOD CHEMISTRY, [Online] vol. 8, no. 11, 2009, pages 1091-1098, XP002552637 ISSN: 1579-4377 Retrieved from the Internet: URL:http://ejeafche.uvigo.es/index.php?opt ion=com_docman&task=doc_view&gid=556> [retrieved on 2009-10-21]
- RAOUF GA ET AL.,: "FT-IR spectroscopy as a tool for identification of apoptosis-induced structural changes in A549 cells treated with PM 701" INTERNATIONAL JOURNAL OF OF NANO BIOMATERIALS (ARTICLE ABSTRACT), [Online] 2009, XP002552638 Retrieved from the Internet: URL:http://www.inderscience.com/search/ind ex.php?action=record&rec_id=27736&prevQuer y=&ps=10&m=or> [retrieved on 2009-10-21]
- MURA U ET AL: "PURINE SALVAGE AS METABOLITE AND ENERGY SAVING MECHANISM IN CAMELUS-DROMEDARIUS THE RECOVERY OF GUANINE", COMPARATIVE BIOCHEMISTRY AND PHYSIOLOGY B, vol. 87, no. 1, 1987, pages 157-160, XP025794571, ISSN: 0305-0491
- MUSUMECI M L ET AL: "Use of a topic containing urea with keratolytic activity in psoriasis", DERMATOLOGIA CLINICA, CENTRO ITALIANO CONGRESSI, ROME, IT, vol. 24, no. 1-2, 1 January 2004 (2004-01-01), pages 16-18, XP009152085, ISSN: 0392-1395
- DATABASE EMBASE [Online] ELSEVIER SCIENCE PUBLISHERS, AMSTERDAM, NL 1963 MARTINSON E.E. ET AL: 'The trophic effect of urea and its use in the treatment of neurotrophic skin diseases (russian)' Database accession no. EMB-0007733174 & MARTINSON E E ET AL: "The trophic effect of urea and its use in the treatment of neurotrophic skin diseases (russian)", VESTN.DERM. VENER. 1963, vol. 7, 1963, pages 2-22,

## Description

### BACKGROUND OF THE INVENTION

### 1. FIELD OF THE INVENTION

The present invention relates to an absolutely novel use of camel urine for medicinal purposes and also relates to the preparation of it by lyophilization as a drug that called PM701 or fractionating it to active fractions that are coded as PMF and PMFK. More specifically, the present invention relates to isolating the bio-active fraction PMF and the most effective subfraction PMFK from the lyophilized PM701 of the adult single-humped Arabian camel, species Camelus dromedarius and the absolutely novel use of these bio-active fractions as a selective anti-cancer agent. The fractionation will be implicated in reducing the dosage of the whole lyophilized urine and increasing its efficacy. The invention also relates to the preparation of a novel pharmaceutical composition (capsules, syrup, ointment and jell....etc) comprising an effective amount of the bio-active fraction PMF or the bioactive subfraction in a composition of 435mg/each capsule or teaspoonful with a range 7 or 5 capsules or teaspoonful /day respectively, which are targeted to the human cancer tissues through their anti-proliferative and apoptotic activities without harming the other normal tissues.

### 2. DESCRIPTION OF THE RELATED ART

Cancers are uncontrolled cell proliferations that result from the accumulation of genetic changes in cells endowed with proliferative potential. After a variable latency period during which they are clinically silent, the malignant cells progress to aggressive invasive and metastatic stages with tumor formation and wide-spread dissemination throughout the body.

Despite important advances in treatment, cancers still account for 28% of death in Western countries, and more than this ratio in other countries. Treatment of cancer has relied mainly on surgery, chemotherapy, radiotherapy and more recently immunotherapy. However, for the most frequent types of cancers (lung, breast, colo-rectal and the leukemias) complete remission and cure has not been achieved. Therefore, the development of new approaches for treating cancer patients is highly desirable and critically needed particularly for those patients whose disease has progressed to a metastatic stage and are refractory to standard chemotherapy.

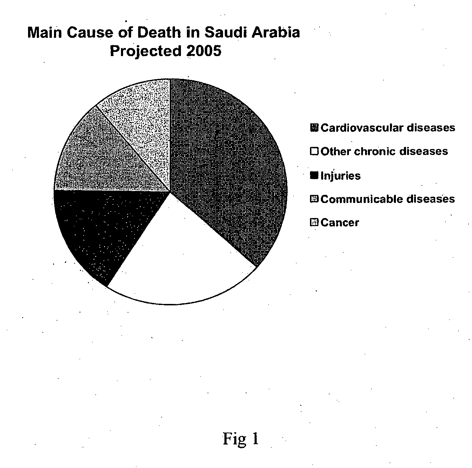

The world health organization published that cancer is a leading cause of death worldwide. From a total of 58 million deaths worldwide in 2005, cancer accounts for 7.6 million (or 13%) of all deaths, in 2005 cancer killed approximately 12,000 people in Saudi Arabia, as shown in FIG. 1, with 8,000 of those people were under the age of 70.

In Prophet Mohamed Medicine (peace be upon him), camel urine is suggested for drinking to improve some symptoms mainly associated with tumor formation in the body. Therefore, camel urine has been used in the Islamic and Arab world, sold and distributed in different size bottles. But it is never scientifically tested. The applicant considered it is worthwhile to scientifically look at this and define its value through in vitro and in vivo assays. The applicant has developed the curiosity about it and asked a number of questions: What possible importance can camel urine have? Whether the component camel urine is having any activity on tumor formation by itself or some of its parts? Does the fresh urine have any effects on human cancer tissues? Could one formulate this urine in a convenience form for human use? Does the formulation of urine enhance its activity or reduce it? Dose the camel urine contain microorganism? Is it safe to use it as a drug or does it have toxicological effects on human diverse organs.

In addition, could one fractionate the lyophilized camel urine in order to separate the bio-active fractions? To which part of the cell does the camel urine react? What are the biochemical, biophysical, and biological evidences for its efficacy?

Camel's urine can be considered as an effective animal origin substance/ secretion with the capacity of improvement of some symptoms mainly associated with tumors formation in the body but it does need substantiation through scientific experimentation. Thus, the applicant considered it worthwhile to scientifically look at this and define its values through in vitro and in vivo assays. The applicant in the first instance probed whether it contained anti-cancer agent since such a property would make it a highly useful natural substance. In related art, use of 'piperine' as a bioavailability enhancer has been described in United States Patent 5616593 and 5972382.

Also cow's urine distillate or a dried fraction used for improving activity and bioavailability of antibiotics drugs as described in later patent United States Patent 6896907.

Patent NZ522665-A (30.04.2004) describes a cell differentiation agent-II (CDA-II), purified from fresh human urine, that is effective for the therapy and prevention of cancer.

M.M. Al-Harbi et al., Journal of Ethnopharmacology 52 (1996) 129-137, describe the cytotoxic effect of camel urine in the bone marrow cells of mice which is comparable with that of standard drug cyclophosphamide. Camel urine is, however, non-clastogenic possibly due to the presence of antioxidant and antimutagenic compounds.

### BRIEF SUMMARY OF THE INVENTION

Some of the above questions have been solved and addressed herein. To answer the first set of questions the applicant collected the camel urine (PM70 1) from natural pastures in various areas within Jeddah, Makah, Madinah and Riyadh governorate (Saudi Arabia) in different size bottles at any time of the day. The urine was tested on CLED media and blood agar in sterile condition and did not notice any growth of microorganism. Tissue cultures of human cancer tissues and normal tissues were used in studying the effect of (camel urine) PM701 on the behavior of cancer cells and normal cells. PM701 appears to target the cancer cells and have anti-proliferative, apoptotic efficacy on them.

Surprisingly, the same PM701 exhibited nourishing effects on normal healthy cells; this implies that PM701 have a selectively killing effect on cancer cells and reparative effect on normal dividing cells, these results leading to this invention. The novelty of the invention lies in the fact revealed through precise experimentation that the PM701 action and its effectiveness are achievable only in the range of concentration which is literally in nano to micro-gram levels. That should be the reason for detection such a valuable potential of PM701 in targeted to the cancer cells. The utilized of fresh PM701 remains non-acceptable and non-convenience for human use therefore, the applicant also further lyophilized the liquid PM701 to obtain 0.2g/ml of powder. we re-examined the lyophilized PM701 on normal cells and diverse cancer cells in both cell culture and animal models, which showed the same anti-cancer efficacy and that was mediated by apoptosis as determined by an MTT test and electron microscopy examination.

The applicant thought of utilizing PM701 as an alternative drug for cancer therapy since it showed a target effect on cancer cells and no side effects on the normal tissues, but the amount of lyophilized PM701 dosage per day was as a load in the body (46 capsules/day), ultimately leading to use one of the ways, which has been feasible for drastically reducing the daily dosage of this anti-cancer agent PM701 and increasing the efficiency of the dosage activity too and has also high commercial importance. Therefore the bio-guided fractionation approach was used with lyophilized PM701, which lead us to the isolation and identification of the bio-active fractions, which is responsible for the anti-cancer efficacy observed with the whole urine.

For the purpose of the present invention the following terms are defined below.

The term "anti-cancer therapy" is intended to mean growth inhibition/eradication of primary tumors, stabilization of tumor growth, inhibition of metastasis formation, or prevention of tumor formation. Furthermore, anticancer activity also covers any combination between our substances and other known or investigational anticancer agents, in order to improve the therapeutic efficacy of drugs.

The main purpose of this work is to reach an optimum alternative drug for cancer treatment other than radiation or chemotherapy.

A mainstream approach is treating cancer with new methods other than chemotherapy and radiotherapy, which have very bad side effects on normal tissues.

Another objective of the invention is to provide new use of the PM701 as a selective anti-cancer agent.

In another objective of the invention is to provide a method for improving activity of PM701 via its bio-active fractions.

Also another objective of the invention is to provide a process for the isolation of the active fractions form PM701 or camel urine.

Still another objective of the invention is to provide the bio-active fractions of lyophilized PM701 as in an effective amount as a novel pharmaceutical composition (capsules, syrup, ointment and jell....ect).

The important objective of the invention is to prevent destruction of normal tissues during the process of cancer treatment. To reach a protocol for treating cancer patients with available substances and at a low cost.

The invention relates to a new use of known abundantly available camel urine as anti-cancer agent and to provide the bio-active fractions of it that be useful in cancer treatment. In accordance with one aspect of the invention there is provided a novel anti-cancer pharmaceutical composition comprising an acceptable, effective anti-cancer amount of bio-active fractions of PM701. The invented bio-active fractions targeted the cancer tissues without any side effects on the normal tissues.

### BRIEF DESCRIPTION OF THE SEVERAL VIEWS OF THE DRAWINGS

FIG. 1 is a chart of projected causes of death in Saudi Arabia.
FIG. 2 illustrates the chromatographic subfractionation of the organic soluble fraction PMF (G) led to the isolation of 7 subfractions referred to as G1-G7.
FIG. 3A illustrates a curve of the in vitro cytotoxic effect of lyophilized PM701 using human lung carcinoma cell line, A549 in different incubated periods comparing with non treated cancer cells.
FIG. 3B illustrates a curve of the in vitro non-cytotoxic effect of lyophilized PM701 using vero cell line in different incubated periods comparing with non treated normal cells.
FIG. 3C illustrates the in vitro cytotoxic effect of lyophilized PM701 on the cell morphology of human lung carcinoma cell line, A549 (a) in PM 701. Note the damage of cells as compared with the control cells that were incubated in MEM media (b) (40X).
FIG. 4A illustrates a curve of the in vitro cytotoxic effect of PMF fraction using human lung carcinoma cell line, A549 in different incubated periods comparing with non treated cancer cells.
FIG. 4B illustrates a curve of the in vitro non-cytotoxic effect of PMF fraction using normal human foreskin cell line, HFS in different incubated periods comparing with non treated normal cells.
FIG. 4C illustrates the in vitro cytotoxic effect of PMF fraction on the cell morphology of human lung carcinoma cell line (A549), a. treated cells; b. non treated cells (40X).
FIG. 5A illustrates a curve of the in vitro cytotoxic effect of PMFK subfraction using human lung carcinoma cell line, A549 in different incubated periods comparing with non treated cancer cells.
FIG. 5B illustrates a curve of the in vitro non-cytotoxic effect of PMFK subfraction using normal human foreskin cell line, HFS in different incubated periods comparing with non treated normal cells.
FIG. 5C illustrates the in vitro cytotoxic effect of PMFK subfraction on the cell morphology of human lung carcinoma cell line (A549), a. treated cells; b. non treated cells (20X).
FIG. 6 illustrates ultrastructural morphological hallmarks features that characterize apoptosis, as shown by chromatin condensation and membrane blebbing in treated cancer cells with PM701.
FIG. 7A illustrates the in vitro cytotoxic effect of -2, -3 and -4 concentration of PM701 using mice leukemia cells, L1210.
FIG. 7B illustrates the effect of PM 701 on the cell morphology of mice leukemia cells, L1210, a- treated cells; b- non treated cells - note the normal cell size (arrows) (40X).
FIG. 8A illustrates an MTT test show the effect of two different concentrations of PM701: -2 or high and -3 or low on four different cell densities 1, 3, 6 and 10 x 10³ cell/ well.
FIG. 8B illustrates an MTT test shows the effect of PM701 on two types of carcinogenic cells A549 and L1210 at 3 x 10³/well.
FIG. 9 illustrates schematically the process of fractionation and subfractionation of PM 701.

### DETAILED DESCRIPTION OF THE INVENTION

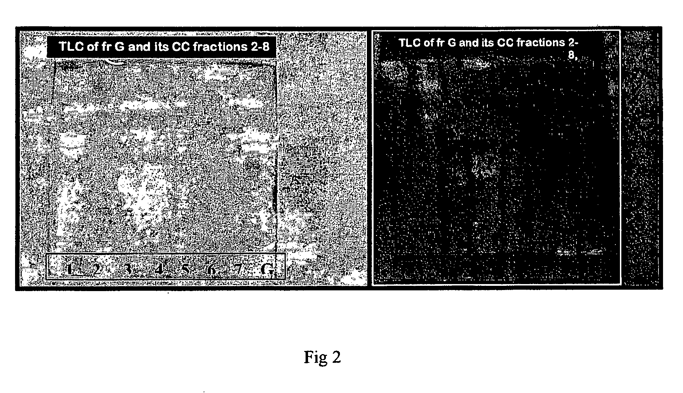

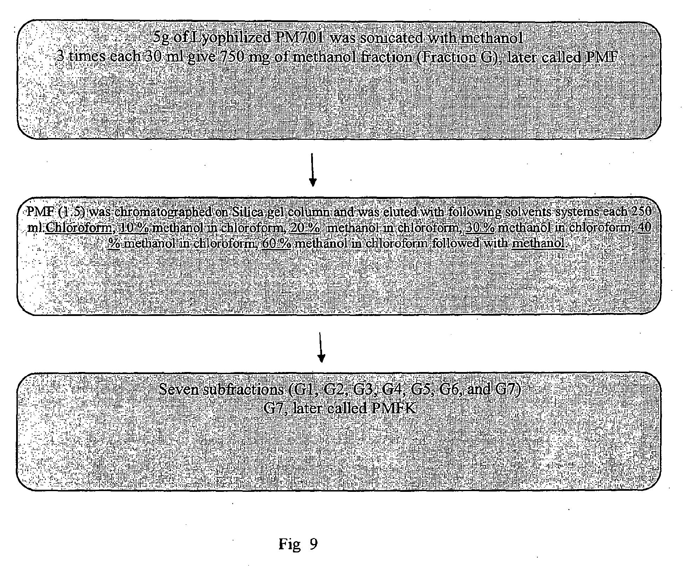

As shown in FIGS. 2-9, the present invention solves the problem of searching for and obtaining a plentifully, available, cheap, and natural material (PM701) as an anti-cancer agent with a higher and selective potency on cancer cells, obtained from camel urine. Additionally, the bio-active fraction of PM701 was isolated, which is coded PMF due to its highly selectively and highly cytotoxic properties on cancer cells, which is responsible on the whole PM701 effect. Furthermore, PMF was subfractionated and led to purification of seven subfractions, the seventh which is coded PMFK has cytotoxic properties on cancer cells as much as PMF cytotoxic properties.

PMF and PMFK have cytotoxic and killing activities in vitro on human lung cancer cell line (A549), mice leukemic cancer cell line (L1210), human colon cancer cells (HCT116), human glioma cancer cells (U251), human breast cancer cells (MCF7), and human liver cancer cells (HepG2) without affecting normal cells such as vero and human foreskin cells HFS. More important were the obvious effects on inhibition the cancer cell division activity through the morphological hallmarks and biochemical features that characterize apoptosis, as shown by loss of cell viability, chromatin condensation, and reducing metabolic activity using an MTT test.

In another embodiment, PM701 was used in vivo in treating animal models that were inoculated with cancer cells; the result of in vivo testing is as satisfactory as in vitro effect at tissue culture level.

In an embodiment of the present invention a pharmaceutical composition comprising an effective amount of PMF or PMFK as bioactive anticancer compounds and pharmaceutically acceptable additives selected from anticancer compounds.

In another embodiment, PMF and PMFK are used as bioavailability anticancer therapy directly or in combination with other anticancer molecules.

In yet another embodiment, PMF and PMFK are induced the apoptosis of cancer cells.

In yet another embodiment, the PM701 fractionation helps us to isolate the bio-active molecules, which act better on the target cancer cells by decreasing its proliferation.

In yet another embodiment, the cancer cells may be lung, leukemia or any cancer cells.

In yet another embodiment, PMF used in vitro in the range between 80 µg/ml to 800 µg/ml and PMFK used in the range between 0.2 µg/ml to 2 µg/ml.

In still another embodiment, the bioactive fractions (PMF and PMFK) enhance the antiproliferative and apoptotic activities of anti-cancer agents (PM701) for 2.8 folds.

The methodology followed by us for this screening included specifically designed in vitro and in vivo bioassays as described below. The cell lines used in this invention were obtained from cell bank of Tissue Culture Unit in King Fahd Medical Research Center (KFMRC).

### DEVELOPMENT OF POWDER FORM

For enhancing the utility and convenience of application of PM70 1, liquid PM701 was lyophilized to reach a solid form. The applicant further fractionated the solid form to obtain the bioactive fraction(s), which is also free of the typical smell of camel urine that it is more readily acceptable to the humans. For this purpose the lyophilized PM701 was fractionated as described by the following procedure:
Step 1: PM701 was collected in the stainless steel container directly from the camel, which is maintained in hygienic environment.
Step 2: 90g of liquid PM701 was added to Microcrystalline cellulose (10 gm). This will give 100 g of a mixture, that frozen at - 80° C. in Pyrex apparatus for 20-24 hrs.
Steps 3: 100g of the mixture is lyophilized in the lyophilizer at room temperature for 5 days to obtain 20 g of solid form PM701.
Step 4: Mixtures leave in a desiccator with calcium chloride, in present of vacuum pressure for one day at room temperature
Step 5: Another method using Spray Dryer machine. This machine makes the material from liquidity to micro-powder
Step 6: The powder PM701 is packed in fridge in a sterilized glass container for further use.

### FRACTIONATION OF A LYOPHILIZED PM701 SEPARATION OF ACTIVE INGREDIENTS

The following steps were performed.

### i. Solvent extraction method for fractionation

Step 1: A sample of 5mg of lyophilized PM701 was sonicated with methanol three times each 30 ml to give about 750mg of methanol fraction, which is called (G).

### ii. Molecular sieving method for subfractionation

Step 1: Subfractionation of PMF using column of different type of gel with different sieving capacity (e.g. Sephadex LH-20, Sephadex-25, -50, ..).

Step 2: A sample of 1.5 mg of The methanol fraction (G) was chromatographed on Silica gel column and was eluted with following solvents systems each 250 ml, chloroform, 10 % methanol in chloroform, 20 % methanol in chloroform, 30 % methanol in chloroform, 40 % methanol in chloroform, 60 % methanol in chloroform followed with methanol.

Step 3: The seven subfractions are purified and individual subfractions are separated by high-performance liquid chromatography.

Step 4: All subfractions, which comes out of column were tested for similar activity as that of PM701 at the tissue culture level.

The organic solvent soluble fractions and subfractions were found to have a strong antiproliferative activity in a panel of human cancer cell lines derived from lung, breast, colon, brain, liver and leukemia. In vitro, the fractions and subfractions demonstrate antiproliferative and antiapoptotic activities in tissue culture experiments.

### ASSAY FOR IN VITRO DOSE DETERMINATION

a. The optimum inhibitory concentration of PM701 is evaluated and determined against different cancer cells, human lung cancer cells (A549), mice leukemic cells (L1012), human colon cancer cells (HCT116), human glioma cancer cells (U251), human breast cancer cells (MCF7), and human liver cancer cells (HepG2), through the in vitro tissue culture experiments.
b. The optimum inhibitory concentration of PM701 showing a cytotoxic effect on cancer cells while there is no any cytotoxic effect on normal cells, suggesting that PM701 can act selectively on cancer cells while nourishing normal cells.
   1 ml of PM701 or PMF or PMFK dissolved in 10 ml standard media, which is called -1 (high).
   1 ml of PM701 or PMF or PMFK dissolved in 100 ml standard media, which is called - 2
   1 ml of PM701 or PMF or PMFK dissolved in 1,000 ml standard media, which is called - 3 (mid).
   1 ml of PM701 or PMF or PMFK dissolved in 10,000 ml standard media, which is called - 4.
   1 ml of PM701 or PMF or PMFK dissolved in 100,000 ml standard media, which is called - 5 (low).

The in vitro experiments showed that the best effect observed when used mid (-2 and -3) concentrations, so we fixed the in vitro and in vivo doses using these medium concentration.

### IN VITRO ANTIPROLIFERATIVE ACTIVITY ASSAY (CELL CULTURE)

### Cell Lines and cell culture

1. Lung cancer cells (A549), human colon cancer cells (HCT116), human glioma cancer cells (U251), human breast cancer cells (MCF7), and human liver cancer cells (HepG2), commercial cell lines obtained from King Fahd Medical Research Center (KFMRC) is inoculated at a density of about 0.5×10⁵ cells in MEM medium in the wells of 24 well plate. L1210 cells were prepared by the same way in RPMI (1640) supplemented with 10% heat-inactivated fetal calf serum.
2. This is replaced with fresh medium after 24 hours in each well.
3. The test component(s) is added at desired concentrations in different wells just after the medium replacement.
4. Observations are recorded and the cell count after 0, 24, 48, and 72 hours for which the following steps are required.
   a. The medium is removed from the wells.
   b. The wells are rinsed with 1 ml PBS (Phosphate buffer saline).
   c. To each well 500 µl of freshly prepared trypsin (0.1% in PBS) solution is added.
   d. Typsin solution is removed after 30 seconds and the plate is gently tapped till the cells are released from the plate surface.
   e. Fresh 1 ml of growth medium is added and agitated with a pipette to obtain a cell suspension.
   f. Cell suspension was prepared (1:1): 20 ml of cells with 20 ml of (0.4%) Trypan blue, 10 µl of cell suspension is taken on the haemocytometer and a cover glass is placed over the counting chamber.
   g. The number of viable cells is counted in 5 big squares and the readings are taken from 5 microscopic fields to determine the average.
   h. The cell count (titer per ml) in the original sample is then calculated as average count×10⁴.

### COMPOSITION OF MINIMUM ESSENTIAL MEDIUM (MEM)

MEM powder (ICN) = 9.95g, NaHCO3 (Powder) = 2.2 g, glutamine (Powder) = 0.3 g L, Non essential amino acid (100X) = 10 ml, Hepes (100X) solution = 10 ml, antibiotic mix (Penicillin + Streptomycin) = 10 ml, deionzed-distilled water = 1 liter.

Stirrer for 1 hrs at room temperature, PH (6.8-7.4).

Sterile-filtered through a 0.22 µm filter and stored at + 4° C.

### CYTOTOXICITY ASSAY

The MTT test is used for determination of the cytotoxicity or the anticarcinogenic effects of PM701 on two types of cancer cells, A549 and L1210. This test measures the cell viability as a percentage of control untreated cells.

An MTT assay was performed to evaluate the growth effects of PM 701. The MTT assay is colorimetric assay based on the tetrazolium salt MTT that detects cell viability. Dissolved MTT is converted to an insoluble purple formazan by cleavage of the tetrazolium ring by dehydrogenase enzymes in living but not dead cells.

MTT was dissolved in phosphate buffered saline (PBS) at 5 mg/ml and filtered through a 0.22 µm filter to sterilize and remove the small amount of insoluble residue then stored at 2-8° C for frequent use. Stock solution of MTT is added to each culture being assayed to equal one tenth the original culture volume. Using isopropanol is measured by spectrophotometricaly yielding absorbance as a function of concentration of converted dye. Exponentially growing cells (3 ×10³ cells/100 µl) were seeded in 96-well plates and incubated for 24 h. Cells were then treated continuously with the various fractions and subfractions. At a selected time, 10 µl of stock MTT solution was added to all wells for the assay. After a further period of incubation (4 hours), the medium was aspirated from the wells as completely as possible without disturbing the formazan crystals. Then, 100 µl of isopropanol is added to each well for dissolving the resulting precipitate. The concentration of the dye is then measured at 570 nm on plate reader (Microplate Reader Model 450; Bio-Rad). The optical density obtained is directly related to the viability of cells.

The MTT assay distinguishes between viable and non-viable cells on the basis that physiologically active mitochondria metabolizes the MTT only in viable cells. The IC50 was calculated as the concentration of drug causing a 50 % inhibition in the absorbance compared to cells treated with solvent alone.

### RESULTS

Methanol soluble fraction (PMF) but not water soluble fractions, was found to have a potent antiproliferative activity in A549, L1210 and other cancer cell lines. Further chromatographic subfractionation of these organic soluble extract led to the isolation of 7 fractions referred to as G1-G7, as shown in FIG. 2 which illustrates the chromatographic subfractionation of the organic soluble fraction PMF led to the isolation of 7 subfractions referred to as G1-G7. Solvent system: CHC13-MeOH-Water 65: 35: 6. Spray Reagent: P-anisaldehyde Reagent, heating at 110°C for 5 min.

PM 701, PMF, and PMFK showed highly cytotoxic effect on cancer cells compared with non- cytotoxic effect on normal cells.

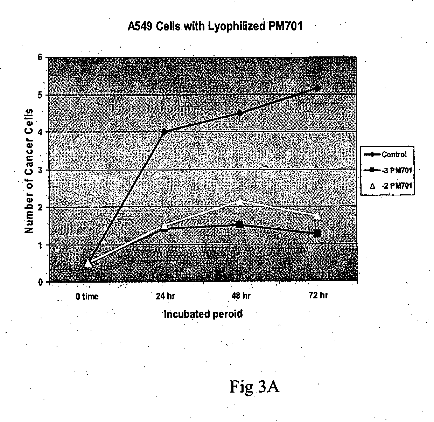

FIG. 3A illustrates a curve of the in vitro cytotoxic effect of lyophilized PM701 using human lung carcinoma cell line, A549 in different incubated periods comparing with non treated cancer cells.

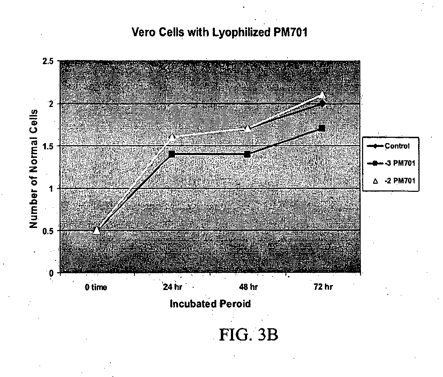

FIG. 3B illustrates a curve of the in vitro non-cytotoxic effect of lyophilized PM701 using vero cell line in different incubated periods comparing with non treated normal cells.

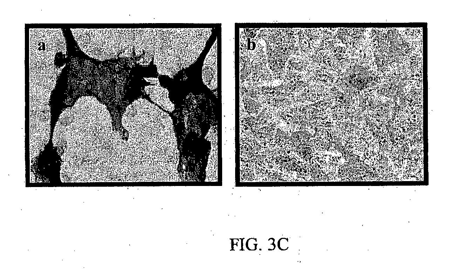

FIG. 3C illustrates the effect of lyophilized PM701 on the cell morphology of human lung carcinoma cell line, A549. Cancer cells A549 imaged (40X) after incubation for 24 h, fixed and stained with Coomassie blue (a) in PM 701. Note the damage of cells as compared with the control cells that were incubated in MEM media (b).

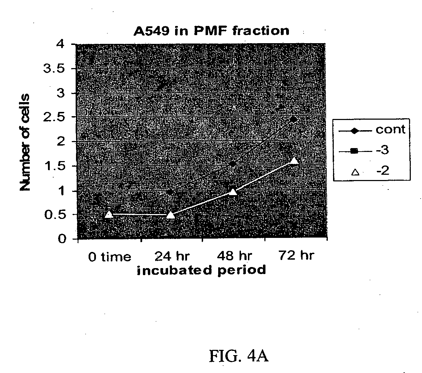

FIG. 4A illustrates a curve of the in vitro cytotoxic effect of PMF fraction using human lung carcinoma cell line, A549 in different incubated periods comparing with non treated cancer cells.

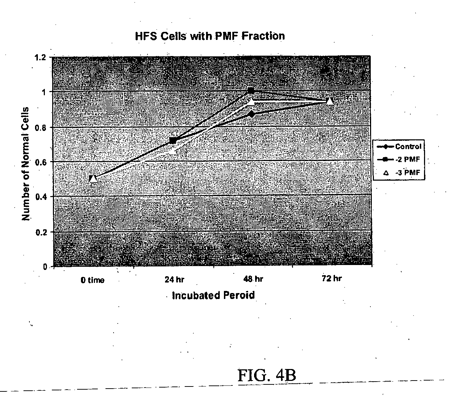

FIG. 4B illustrates a curve of the in vitro non-cytotoxic effect of PMF fraction using human foreskin cell line, HFS in different incubated periods comparing with non treated normal cells.

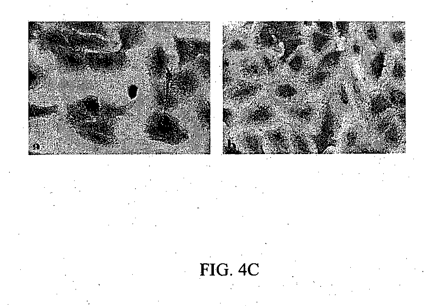

FIG. 4C Illustrates the effect of PMF fraction on the cell morphology of human lung carcinoma cell line, A549, a. treated cells; b. non treated cells (40X).

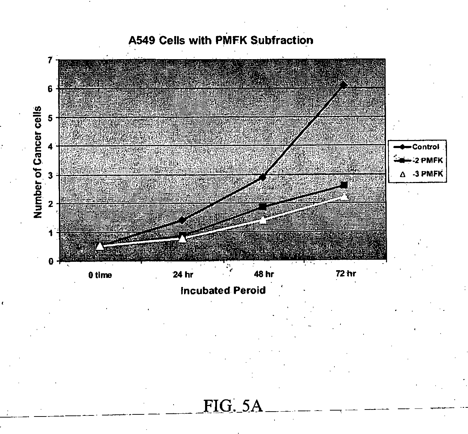

FIG. 5A illustrates a curve of the in vitro cytotoxic effect of PMFK subfraction using human lung carcinoma cell line, A549 in different incubated periods comparing with non treated cancer cells.

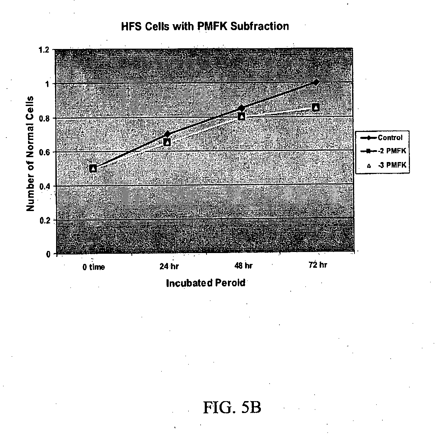

FIG. 5B illustrates a curve of the in vitro non-cytotoxic effect of PMFK subfraction using human foreskin cell line, HFS in different incubated periods comparing with non treated normal cells.

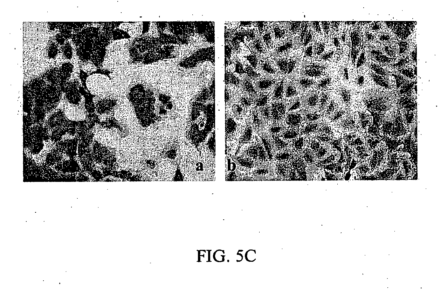

FIG. 5C illustrates the effect of PMFK subfraction on the cell morphology of human lung carcinoma cell line, A549, a. treated cells; b. non treated cells (20X).

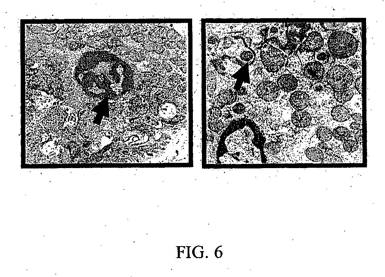

The morphological changes of treated cells with lyophilized PM701 characterize apoptosis, as shown by loss of cell viability, membrane blebbing, and chromatin condensation and shown in FIG 6.

FIG. 6 illustrates ultrastructural morphological hallmarks features that characterize apoptosis, as shown by chromatin condensation and membrane blebbing in treated cells with PM701.

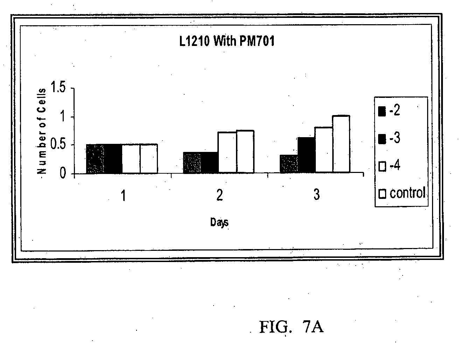

FIG. 7A illustrates the in vitro cytotoxic effect of -2 and -3 concentration of PM701 using mice leukemia cells, L1210.

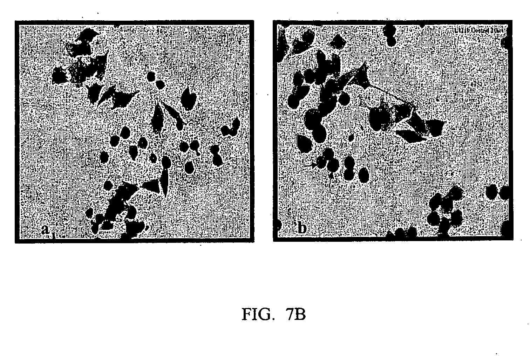

FIG.7B illustrates the effect of PM 701 on the cell morphology of mice leukemia cells, L1210, a. treated cells; b. non treated cells. Note the normal cell size (arrows) (40X).

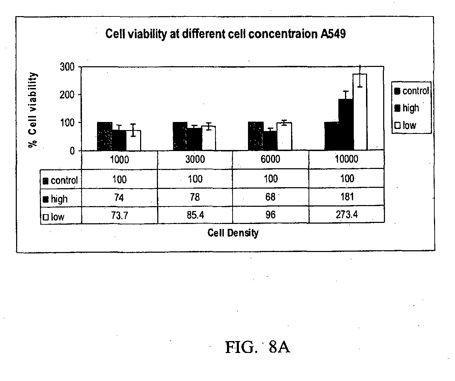

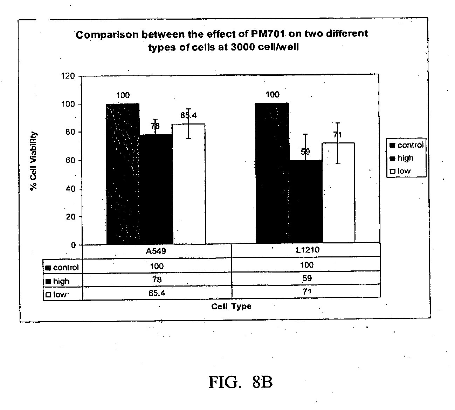

PM 701 reduced metabolic activity of cancer cells using MTT test as shown in FIGS 8A and 8B.

FIG. 8A illustrates an MTT test show the effect of two different concentrations of PM701: -2 or high and -3 or low on four different cell densities 1, 3, 6 and 10 x 103 cell/ well.

FIG. 8B illustrates an MTT test shows the effect of PM701 on two types of carcinogenic cells A549 and L1210 at 3 x 103/well.

Methanol soluble fraction PMF was found to have a good anticancer activity on carcinoma cell lines. A dose relationship was also observed, as shown in FIG. 4A.

## Claims

1. A camel urine fraction (PMF) obtainable according to a method comprising the steps of:
- lyophilizing liquid camel urine (PM701) obtained from the adult single-humped Arabian camel *Camelus dromedarius* in the presence of microcrystalline cellulose; and
- fractionating the lyophilized PM701 by sonication with methanol in order to isolate the bio-active fraction PMF being soluble in methanol.

2. A camel urine subfraction (PMFK) obtainable according to a method comprising the steps of:
- lyophilizing liquid camel urine (PM701) obtained from the adult single-humped Arabian camel *Camelus dromedarius* in the presence of microcrystalline cellulose;
- fractionating the lyophilized PM701 by sonication with methanol in order to isolate the bio-active fraction (PMF) being soluble in methanol; and
- subjecting the isolated PMF to chromatographic subfractioning in solvent systems, each subfraction having a volume of 250 ml solvent consisting essentially of chloroform and methanol to obtain seven subfractions, with the seventh subfraction being called PMFK.

3. The PMF or PMFK of claim 1 or 2, wherein the PM701 is obtained in a powder form by the lyophilization of liquid PM701 in the range of 20g for each 100 ml of fresh liquid PM701.

4. The PMF or PMFK of claim 1 or 2, wherein the dried PM701 of the camel urine lyophilized can be obtained by distillation or by spray dryer.

5. The PMF of claim 1 and the PMFK of claim 2, wherein the dried PMF and PMFK obtained from the camel urine lyophilized is devoid of a camel urine smell.

6. PMF according to claim 1 for use in the treatment of cancer.

7. PMFK according to claim 2 for use in the treatment of cancer.

8. The PMF for use according to claim 6, wherein concentration effects on cell viability and proliferation of cancer cells is at concentrations in the range of 80 µg/ml and 800 µg/ml.

9. The PMFK for use according to claim 7, wherein concentration effects on cell viability and proliferation of cancer cells is at concentrations in the range of 0.2 µg/ml and 2 µg/ml.

10. The PMF for use according to claim 6, comprising an effective amount of the PMF in the amount of 435mg/capsule with 7 capsules/day.

11. The PMFK for use according to claim 7, comprising an effective amount of the composition in the amount of 435mg/capsule with 5 capsules/day.

12. Pharmaceutical composition comprising at least one anticancer agent, and PMF or PMFK according claim 1 or 2.

13. The composition of claim 12, comprising an effective amount of the composition in formulations selected from capsules, syrup, injection, ointment, gel and shampoo.

14. The composition of claims 12 and 13, wherein the composition exhibits a target selective anti-cancer effect in causing apoptotic activities in vitro.

15. The composition of claim 14, wherein the cancer types are selected from human lung cells (A549), mice leukemic cells (L1210), human colon cancer cells (HCT116), human glioma cancer cells (U251), human breast cancer cells (MCF7), and human liver cancer cells (HepG2).

## Patentansprüche

1. Eine Kamelurinfraktion (PMF), die gemäß einem Verfahren erhalten werden kann, das die Schritte umfasst:
- Lyophilisieren von flüssigem Kamelurin (PM701), der von einem ausgewachsenen, einhöckrigen arabischen Kamel *Camelus dromedarius* erhalten wurde, in Anwesenheit von mikrokristalliner Cellulose; und
- Fraktionieren des lyophilisierten PM701 durch Ultraschallbehandlung mit Methanol, um die biologisch aktive Fraktion PMF, die in Methanol löslich ist, zu erhalten.

2. Eine Kamelurinunterfraktion (PMFK), die gemäß einem Verfahren erhalten werden kann, das die Schritte umfasst:
- Lyophilisieren von flüssigem Kamelurin (PM701), der von einem ausgewachsenen, einhöckrigen arabischen Kamel *Camelus dromedarius* erhalten wurde, in Anwesenheit von mikrokristalliner Cellulose;
- Fraktionieren des lyophilisierten PM701 durch Ultraschallbehandlung mit Methanol, um die biologisch aktive Fraktion (PMF), die in Methanol löslich ist, zu erhalten; und
- chromatographisches Subfraktionieren der isolierten PMF in Lösemittelsystemen, wobei jede Unterfraktion ein Volumen von 250 ml Lösemittel im Wesentlichen bestehend aus Chloroform und Methanol hat, um sieben Unterfraktionen zu erhalten, wobei die siebte Unterfraktionen als PMFK bezeichnet wird.

3. Die PMF oder PMFK gemäß Anspruch 1 oder 2, wobei PM701 in Pulverform mittels Lyophilisation des flüssigen PM701 im Bereich von 20 g für jede 100 ml frischen, flüssigen PM701 erhalten wird.

4. Die PMF oder PMFK gemäß Anspruch 1 oder 2, wobei der getrocknete PM701 des lyophilisierten Kamelurins durch Destillation oder mit einem Sprühtrockner erhalten werden kann.

5. Die PMF gemäß Anspruch 1 und die PMFK gemäß Anspruch 2, wobei die getrockneten PMF und PMFK, die aus dem lyophilisierten Kamelurin erhalten wurden, frei von Kameluringeruch sind.

6. PMF gemäß Anspruch 1 zur Verwendung in der Behandlung von Krebs.

7. PMFK gemäß Anspruch 2 zur Verwendung in der Behandlung von Krebs.

8. Die PMF zur Verwendung gemäß Anspruch 6, wobei Konzentrationseffekte auf die Zelllebensfähigkeit und Proliferation von Krebszellen bei Konzentrationen im Bereich von 80 µg/ml und 800 µg/ml liegen.

9. Die PMFK zur Verwendung gemäß Anspruch 7, wobei Konzentrationseffekte auf Zelllebensfähigkeit und Proliferation von Krebszellen bei Konzentrationen im Bereich von 0,2 µg/ml und 2 µg/ml liegen.

10. Die PMF zur Verwendung gemäß Anspruch 6, umfassend eine wirksame Menge von PMF in einer Menge von 435 mg/Kapsel mit 7 Kapseln/Tag.

11. Die PMFK zur Verwendung gemäß Anspruch 7, umfassend eine wirksame Menge der Zusammensetzung in einer Menge von 435 mg/Kapsel mit 5 Kapseln/Tag.

12. Pharmazeutische Zusammensetzung, umfassend mindestens ein Antikrebsmittel und PMF oder PMFK gemäß Anspruch 1 oder 2.

13. Die Zusammensetzung gemäß Anspruch 12, umfassend eine wirksame Menge der Zusammensetzung in Formulierungen ausgewählt aus Kapseln, Sirup, Injektion, Salbe, Gel und Shampoo.

14. Die Zusammensetzung gemäß den Ansprüchen 12 und 13, wobei die Zusammensetzung eine Ziel-selektive Antikrebs-Wirkung zeigt, indem sie in vitro apoptotische Aktivitäten verursacht.

15. Die Zusammensetzung gemäß Anspruch 14, wobei die Krebsarten ausgewählt sind aus humanen Lungenzellen (A549), Mausleukämiezellen (L 1210), humanen Dickdarmkrebszellen (HCT116), humanen Gliomkrebszellen (U251), humanen Brustkrebszellen (MCF7) und humanen Leberkrebszellen (HepG2).

## Revendications

1. Fraction d'urine de chameau (PMF) pouvant être obtenue selon un procédé comprenant les étapes de :
- lyophilisation d'urine de chameau liquide (PM701) provenant du chameau d'Arabie adulte à une seule bosse *Camelus dromedarius* en présence de cellulose microcristalline ; et
- fractionnement de la PM701 lyophilisée par sonication avec du méthanol afin d'isoler la fraction PMF bioactive soluble dans le méthanol.

2. Sous-fraction d'urine de chameau (PMFK) pouvant être obtenue selon un procédé comprenant les étapes de :
- lyophilisation d'urine de chameau liquide (PM701) provenant du chameau d'Arabie adulte à une seule bosse *Camelus dromedarius* en présence de cellulose microcristalline ;
- fractionnement de la PM701 lyophilisée par sonication avec du méthanol afin d'isoler la fraction bioactive (PMF) soluble dans le méthanol ; et
- soumission de la PMF isolée à un sous-fractionnement chromatographique dans des systèmes de solvants, chaque sous-fraction ayant un volume de 250 ml de solvant consistant essentiellement en chloroforme et méthanol pour obtenir sept sous-fractions, la septième sous-fraction étant appelée PMFK.

3. PMF ou PMFK selon la revendication 1 ou 2, dans laquelle la PM701 est obtenue sous une forme pulvérulente suite à la lyophilisation de la PM701 liquide dans la plage de 20 g pour 100 ml de PM701 liquide fraîche.

4. PMF ou PMFK selon la revendication 1 ou 2, dans laquelle la PM701 séchée d'urine de chameau lyophilisée peut être obtenue par distillation ou par séchage par atomisation.

5. PMF selon la revendication 1 et PMFK selon la revendication 2, dans laquelle les PMF et PMFK séchées obtenues à partir de l'urine de chameau lyophilisée sont dépourvues de l'odeur d'urine de chameau.

6. PMF selon la revendication 1 pour l'utilisation dans le traitement du cancer.

7. PMFK selon la revendication 2 pour l'utiisation dans le traitement du cancer.

8. PMF pour son utilisation selon la revendication 6, dans laquelle les effets de la concentration sur la viabilité des cellules et la prolifération des cellules cancéreuses s'exercent à des concentrations dans la plage de 80 µg/ml et 800 µg/ml.

9. PMFK pour son utilisation selon la revendication 7, dans laquelle les effets de la concentration sur la viabilité des cellules et la prolifération des cellules cancéreuses s'exercent à des concentrations dans la plage de 0,2 µg/ml et 2 µg/ml.

10. PMF pour son utilisation selon la revendication 6, comprenant une quantité efficace de PMF à la quantité de 435 mg/capsule à raison de 7 capsules/jour.

11. PMFK pour son utilisation selon la revendication 7, comprenant une quantité efficace de la composition à la quantité de 435 mg/capsule à raison de 5 capsules/jour.

12. Composition pharmaceutique comprenant au moins un agent anticancéreux, et de la PMF ou de la PMFK selon la revendication 1 ou 2.

13. Composition selon la revendication 12, comprenant une quantité efficace de la composition dans des formulations choisies parmi les capsules, le sirop, l'injection, la pommade, le gel et le shampooing.

14. Composition selon les revendications 12 et 13, dans laquelle la composition manifeste un effet anticancéreux sélectif de la cible en induisant des activités apoptotiques in vitro.

15. Composition selon la revendication 14, **caractérisée en ce que** les types de cancers sont choisis parmi les cellules du poumon humain (A549), les cellules de la leucémie murine (L1210), les cellules du cancer du côlon humain (HCT116), les cellules des cancers de type gliome humain (U251), les cellules du cancer du sein humain (MCF7), et les cellules du cancer du foie humain (HepG2).
